# EUROPEAN PATENT APPLICATION

(11) **EP 3 593 841 A1**
(43) Date of publication of application: **15.01.2020**
(21) Application number: 18763730.1
(22) Date of filing: 09.03.2018
(51) Int. Cl.: A61M 5/31, A61M 5/28, A61M 5/50

(54) **SYRINGE ASSEMBLY AND PREFILLED SYRINGE**

(30) Priority: 10.03.2017 JP 2017046200
(71) Applicant: Terumo Kabushiki Kaisha, Tokyo 151-0072 (JP)
(72) Inventor: OKIHARA, Hitoshi, Fujinomiya-shi Shizuoka 418-0004 (JP)
(74) Representative: TBK
(86) International application number: PCT/JP2018/009126
(87) International publication number: WO 2018/164250

(57) **Abstract**

A syringe assembly (20) of a prefilled syringe (10) includes a syringe barrel (12) having at a distal end thereof a cylindrical portion (26) configured as a female luer to which a male luer (102) is connectable, and a cap (18). A cap cover (36) of the cap (18) includes a cylindrical body (37), a locking portion (38), and connecting portions (40). The cylindrical portion (26) is provided, on an outer peripheral surface thereof, with protruding portions (28) for restricting movement of the locking portion (38) in a distal end direction with respect to the cylindrical portion (26). A distance (L1) from a distal end of the cylindrical portion (26) to a distal end of each protruding portion (28) in an axial direction of the cylindrical portion (26) is set in the range of 5 mm to 10 mm.

## Description

### Technical Field

The present invention relates to a syringe assembly and a prefilled syringe that include a syringe barrel provided at its distal end with a cylindrical portion configured as a female luer to which a male luer can be connected, and a cap attached to the syringe barrel.

### Background Art

As such a prefilled syringe, for example, U.S. Pat. No. 4,172,457 discloses one with a cap sealing an opening of a cylindrical portion as a female luer provided at the distal end of a syringe barrel filled with a powder in advance. The cap is held by a flange provided at the distal end of the cylindrical portion.

### Summary of Invention

However, the prefilled syringe as in U.S. Pat. No. 4,172,457 described above is not provided with means for discriminating between an unopened state of the cap and a recapped state in which the cap once removed from the syringe barrel is reattached to the syringe barrel. Thus, it is not easy to discriminate between the unopened state and the recapped state of the cap.

The present invention has been made in view of this problem. It is an object of the present invention to provide a syringe assembly and a prefilled syringe that allow secure connection of a male luer, and allow easy and reliable discrimination between an unopened state and a recapped state of a cap.

To attain the above object, a syringe assembly according to the present invention includes a syringe barrel having at a distal end thereof a cylindrical portion configured as a female luer to which a male luer is connectable, and a cap attached to the syringe barrel. The cap includes a cylindrical cap cover, and a plug fixed inside the cap cover, for liquid-tightly sealing an opening of the cylindrical portion. The cap cover includes a cylindrical body to which the plug is fixed, for covering the cylindrical portion, a locking portion provided on a proximal end side of the cylindrical body, and connecting portions connecting the cylindrical body and the locking portion in a breakable manner. The cylindrical portion is provided, on an outer peripheral surface thereof, with at least one protruding portion for restricting movement of the locking portion in a distal end direction with respect to the cylindrical portion. A distance from a distal end of the cylindrical portion to a distal end of the at least one protruding portion in an axial direction of the cylindrical portion is set in a range of 5 mm to 10 mm. The cylindrical body and the plug are removed from the syringe barrel by breaking the connecting portions.

According to this configuration, the connecting portions are broken when the cap is opened. Specifically, in an unopened state of the cap, the connecting portions are not broken, and in a recapped state in which the cylindrical body and the plug once removed from the cylindrical portion are reattached to the cylindrical portion, the connecting portions are broken. This allows the user to easily and reliably discriminate between the unopened state and the recapped state of the cap. Further, since the distance from the distal end of the cylindrical portion to the distal end of the at least one protruding portion in the axial direction of the cylindrical portion is set in the range of 5 mm to 10 mm, the at least one protruding portion can be prevented from being an obstacle when the male luer is connected to the cylindrical portion configured as the female luer. Consequently, the male luer can be securely connected to the cylindrical portion.

In the syringe assembly, the at least one protruding portion may be located between the cylindrical body and the locking portion.

According to this configuration, when the cylindrical body is displaced relative to the cylindrical portion in the distal end direction, the locking portion comes into contact with the at least one protruding portion, so that the connecting portions can be easily broken.

In the syringe assembly, the at least one protruding portion may include two protruding portions provided at positions 180° out of phase in a circumferential direction of the cylindrical portion, and the locking portion may be formed in an annular shape, and have an inner diameter smaller than a distance between protruding ends of the protruding portions in a radial direction of the cylindrical portion.

According to this configuration, the locking portion can be securely brought into contact with the protruding portions when the cap body is removed from the cylindrical portion.

In the syringe assembly, the connecting portions may be provided between the two protruding portions in the circumferential direction of the cylindrical portion.

According to this configuration, interference between the connecting portions and the protruding portions can be avoided.

In the syringe assembly, the cylindrical portion may be provided with an annular portion on the outer peripheral surface thereof, and the at least one protruding portion may extend from the annular portion.

According to this configuration, the annular portion can improve the rigidity of the cylindrical portion as the female luer, and can improve the rigidity of the at least one protruding portion.

In the syringe assembly, the locking portion may have an inner diameter larger than an outer diameter of the annular portion.

According to this configuration, at the time of production of the syringe assembly, the locking portion of the cap can be easily fitted on the proximal end side of the cylindrical portion beyond the at least one protruding portion.

In the syringe assembly, the connecting portions may be provided in the circumferential direction of the cap cover.

According to this configuration, it can be facilitated to visually recognize whether the connecting portions are broken.

In the syringe assembly, the connecting portions may have a thickness dimension along a radial direction of the cap cover smaller than a thickness dimension of a side wall of the cylindrical body.

According to this configuration, the user can break the connecting portions with a moderate force.

In the syringe assembly, the cylindrical body may have a female thread formed on an inner peripheral surface thereof, and the cylindrical portion may have a male thread provided on the distal end side of the outer peripheral surface thereof and screwed to the female thread.

According to this configuration, by rotating the cylindrical body relative to the cylindrical portion with the male thread and the female thread screwed together, the cylindrical body can be easily displaced in the distal end direction relative to the cylindrical portion.

In the syringe assembly, the locking portion may have a restriction portion that is engaged with the at least one protruding portion in the circumferential direction of the cap cover, for restricting movement of the locking portion in a rotational direction with respect to the cylindrical portion.

According to this configuration, by rotating the cap cover in the circumferential direction with respect to the cylindrical portion, the at least one protruding portion and the restriction portion are engaged with each other, so that shearing forces in the circumferential direction can be applied to the connecting portions. Thus, the connecting portions can be easily broken.

In the syringe assembly, the cap may be attached to the syringe barrel only by engagement of the locking portion with the at least one protruding portion, in an unopened state of the cap.

According to this configuration, by the connecting portions being broken at the time of opening the cap, the cylindrical body and the plug are separated from the locking portion. Thus, the cylindrical body and the plug can be prevented from being recapped to the cylindrical portion.

In the syringe assembly, the cap may have a guide for guiding the cylindrical body in the distal end direction when the cylindrical body is rotated relative to the cylindrical portion.

According to this configuration, by rotating the cylindrical body relative to the cylindrical portion, the cylindrical body can be easily displaced in the distal end direction relative to the cylindrical portion.

A prefilled syringe according to the present invention includes the above-described syringe assembly, a medicine with which the syringe barrel of the syringe assembly is filled, and a gasket slidable in the syringe barrel liquid-tightly in an axial direction.

According to this configuration, it is possible to obtain a prefilled syringe having the same function and effect as the above-described syringe assembly.

According to the present invention, in the unopened state of the cap, the connecting portions are not broken, and in the recapped state in which the cylindrical body and the plug once removed from the cylindrical portion are reattached to the cylindrical portion, the connecting portions are broken. Thus, the unopened state and the recapped state of the cap can be easily and reliably discriminated. Further, since the distance from the distal end of the cylindrical portion to the distal end of the at least one protruding portion in the axial direction of the cylindrical portion is set in the range of 5 mm to 10 mm, the male luer can be securely connected to the cylindrical portion.

### Brief Description of Drawings

Fig. 1 is a longitudinal sectional view of a prefilled syringe according to an embodiment of the present invention.
Fig. 2 is an exploded perspective view of the distal end side of the prefilled syringe.
Fig. 3 is an enlarged longitudinal sectional view of the distal end side of the prefilled syringe shown in Fig. 1.
Fig. 4 is a transverse sectional view taken along line IV-IV in Fig. 3.
Fig. 5 is a perspective view of a cap cover constituting the prefilled syringe from a proximal end direction.
Fig. 6A is a first explanatory plan view of an opening operation of a cap, and Fig. 6B is a second explanatory plan view of the opening operation of the cap.
Fig. 7 is a plan view showing a recapped state of the cap.
Fig. 8 is a sectional view showing a state in which a male luer of a different syringe is connected to a cylindrical portion of the prefilled syringe.

### Description of Embodiments

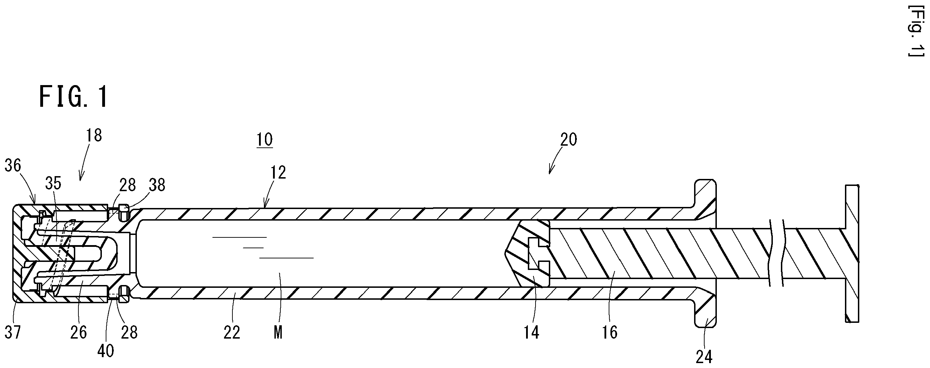

Hereinafter, a preferred embodiment of a syringe assembly and a prefilled syringe according to the present invention will be described with reference to the accompanying drawings. In the following description of the prefilled syringe and its components, the left side in Fig. 1 is referred to as the "distal end," and the right side the "proximal end."

As shown in Fig. 1, a prefilled syringe 10 according to the embodiment of the present invention includes a syringe barrel 12, a medicine M with which the syringe barrel 12 is filled, a gasket 14 slidably inserted in the syringe barrel 12, a plunger 16 connected to the gasket 14, and a cap 18 detachably attached to the syringe barrel 12. In the present embodiment, the syringe barrel 12 and the cap 18 constitute a syringe assembly 20. The prefilled syringe 10 is assembled by inserting the gasket 14 to which the plunger 16 is connected into the syringe barrel 12 of the syringe assembly 20 filled with the medicine M.

The syringe barrel 12 has a cylindrical shape body 22 extending in the axial direction, a flange 24 provided at a proximal end portion of the body 22, and a cylindrical portion 26 protruding from a distal end portion of the body 22 in the distal end direction. The body 22, the flange 24, and the cylindrical portion 26 are integrally formed.

The syringe barrel 12, which is not limited to a particular constituent material, may be formed, for example, of polyolefin such as polypropylene, polyurethane, polyethylene, cyclic polyolefin, or polymethylpentene 1, or a resinous material such as polyester, nylon, polycarbonate, polymethylmethacrylate (PMMA), polyetherimide (PEI), polyethersulfone, polyetheretherketone (PEEK), fluororesin, polyphenylene sulfide (PPS), or polyacetal resin (POM), or a metallic material such as stainless steel, or glass.

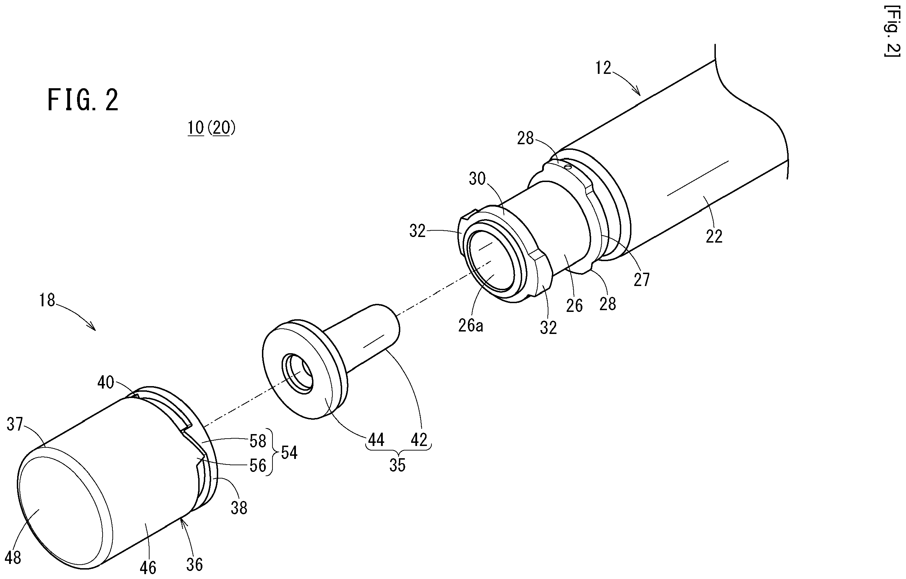

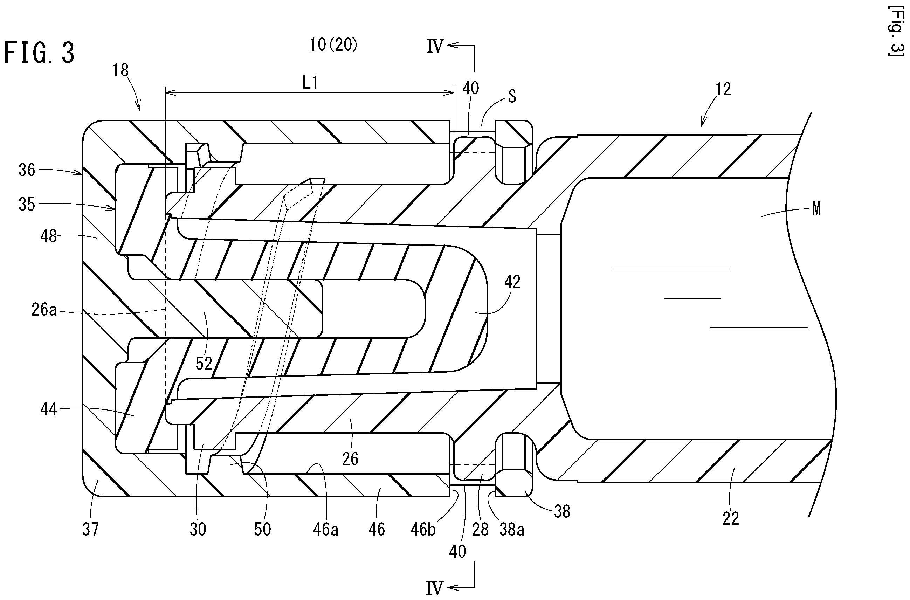

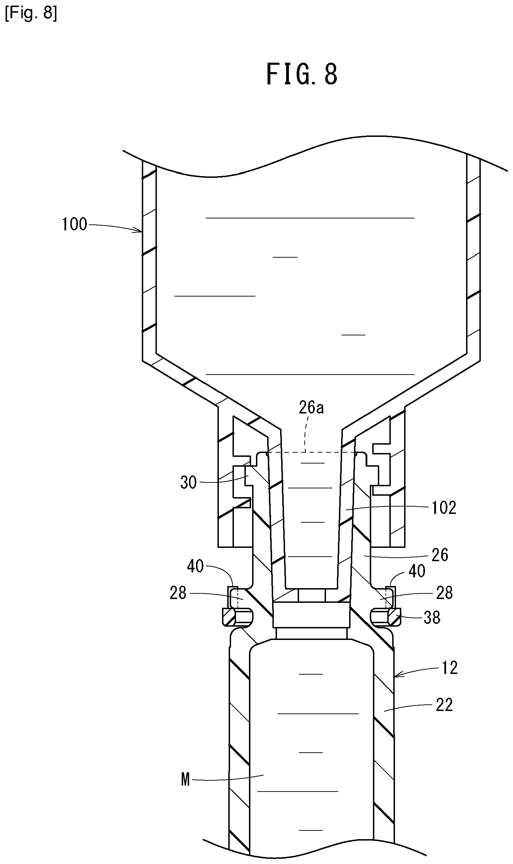

As shown in Figs. 2 and 3, the cylindrical portion 26 is formed in a cylindrical shape as a female luer to which a male luer 102 of a different syringe 100 shown in Fig. 8 is connectable. On the proximal end side of the outer peripheral surface of the cylindrical portion 26, a proximal-end-side annular portion 27 (annular portion) that protrudes radially outward and extends in the circumferential direction of the cylindrical portion 26, and two protruding portions 28 that protrude radially outward from the outer peripheral surface of the proximal-end-side annular portion 27 are provided. The two protruding portions 28 are positioned 180° out of phase in the circumferential direction of the cylindrical portion 26.

The distance L1 from the distal end of the cylindrical portion 26 to the distal end of each protruding portion 28 in the axial direction is set in the range of 5 mm to 10 mm (see Fig. 3). On the distal end side of the outer peripheral surface of the cylindrical portion 26, a distal-end-side annular portion 30 protruding radially outward and extending in the circumferential direction of the cylindrical portion 26, and two male threads 32 protruding radially outward from the outer peripheral surface of the distal-end-side annular portion 30 are provided. The two male threads 32 are positioned 180° out of phase in the circumferential direction of the cylindrical portion 26. The two male threads 32 are 90° out of phase with the two protruding portions 28 in the circumferential direction of the cylindrical portion 26.

The cap 18 has a cylindrical cap cover 36 and a plug 35 fixed inside the cap cover 36 for liquid-tightly sealing an opening 26a of the cylindrical portion 26. The cap cover 36 has a cylindrical body 37 to which the plug 35 is fixed, for covering the cylindrical portion 26, a locking portion 38 provided on the proximal end side of the cylindrical body 37, and two connecting portions 40 connecting the cylindrical body 37 and the locking portion 38 in a breakable manner.

The plug 35 has a cylindrical insertion portion 42 that is open at its distal end and closed at its proximal end and extends in the axial direction of the cap 18, and a sealing portion 44 that surrounds a distal end opening of the insertion portion 42 and protrudes radially outward. The plug 35 is formed in a body without a break between the insertion portion 42 and the sealing portion 44.

Examples of the constituent material of the plug 35 include various rubber materials such as natural rubber, butyl rubber, isoprene rubber, butadiene rubber, styrenebutadiene rubber, and silicone rubber, various thermoplastic elastomers of polyurethane, polyester, polyamide, olefin, styrene, and the like, and compounds of them.

With the cap 18 attached to the cylindrical portion 26, the sealing portion 44 is circumferentially in close contact with a distal end face of the cylindrical portion 26 along the opening 26a of the cylindrical portion 26, and is pressed by a distal end portion of the cylindrical portion 26 in the distal end direction and dented in the distal end direction. Note that a proximal end face of the sealing portion 44 in a natural state (the sealing portion 44 not pressed by the distal end portion of the cylindrical portion 26) is flat.

The cylindrical body 37 is a cylindrical member for covering the cylindrical portion 26. The cylindrical body 37 includes a cylindrical side wall 46 and a distal end wall 48 closing a distal-end opening of the side wall 46, and is formed of a material harder than that of the plug 35 (for example, one of those illustrated as the constituent material of the syringe barrel 12). The proximal end of the cylindrical body 37 is in proximity to the protruding portions 28 with the cap 18 attached to the cylindrical portion 26 (see Fig. 3).

On an inner peripheral surface 46a of the side wall 46 of the cylindrical body 37, a female thread 50 screwed to the male thread 32 provided on the cylindrical portion 26 is formed. By rotating the cylindrical body 37 relative to the cylindrical portion 26 with the female thread 50 and the male thread 32 screwed to each other, the cylindrical body 37 is displaced in the axial direction relative to the cylindrical portion 26. The sealing portion 44 of the plug 35 is supported on an inner surface of the distal end wall 48 of the cylindrical body 37. At the center of the inner surface of the distal end wall 48 of the cylindrical body 37, an anti-rotation portion 52 is provided which protrudes in the proximal end direction along the axis of the cap 18, and contacts an inner peripheral surface of the insertion portion 42 in a state in which it is inserted in the insertion portion 42.

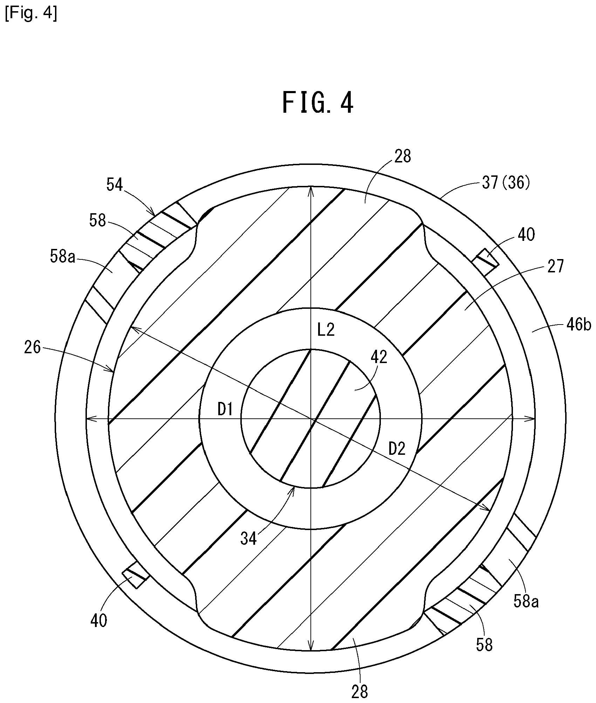

As shown in Figs. 3 and 4, the locking portion 38 is formed in an annular shape, and is disposed in a space S between the protruding portions 28 and the body 22. The inner diameter D1 of the locking portion 38 is smaller than the inner diameter of the side wall 46 of the cap cover 36 and the outer diameter of the body 22. The outer diameter of the locking portion 38 is substantially the same as the outer diameter of the side wall 46 of the cap cover 36. In other words, the inner diameter D1 of the locking portion 38 is larger than the outer diameter D2 of the proximal-end-side annular portion 27 and smaller than the distance L2 between the protruding ends of the two protruding portions 28. That is, the movement of the locking portion 38 in the distal end direction with respect to the cylindrical portion 26 is restricted by the protruding portions 28, and the movement in the proximal end direction with respect to the cylindrical portion 26 is restricted by the body 22.

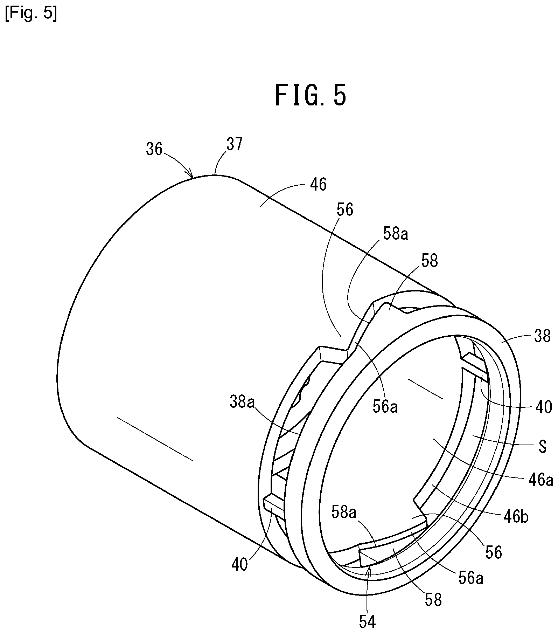

In Figs. 4 and 5, the two connecting portions 40 are provided at positions 180° out of phase in the circumferential direction of the cap cover 36. That is, the connecting portions 40 are provided between the protruding portions 28 in the circumferential direction of the cylindrical portion 26. The connecting portions 40 connect a proximal end face 46b of the side wall 46 of the cylindrical body 37 and a distal end face 38a of the locking portion 38 to each other with space therebetween in a breakable manner. The protruding portions 28 are located between the cylindrical body 37 and the locking portion 38. The thickness dimension of the connecting portions 40 along the radial direction of the cap cover 36 is smaller than the thickness dimension of a proximal end portion of the side wall 46 of the cylindrical body 37 (see Fig. 5).

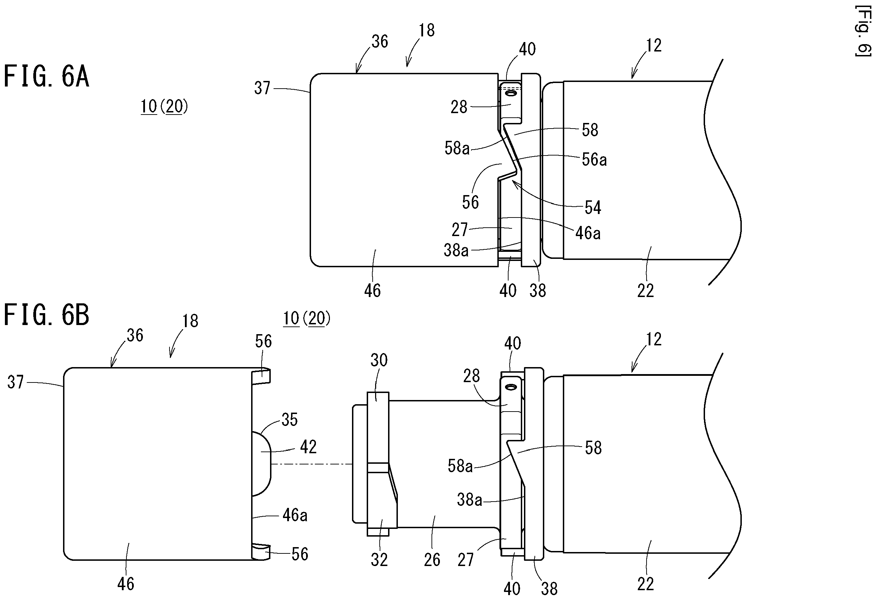

As shown in Figs. 5 and 6A, the cap 18 has a guide 54 for guiding the cap cover 36 in the distal end direction when the cap cover 36 is rotated relative to the cylindrical portion 26. The guide 54 includes two first protrusions 56 protruding in a triangular shape in the proximal end direction from the proximal end face 46b of the side wall 46 of the cylindrical body 37, and two second protrusions 58 protruding in a triangular shape in the distal end direction from the distal end face 38a of the locking portion 38 that faces the proximal end face of the peripheral wall of the cap cover 36.

The two first protrusions 56 are provided at positions 180° out of phase in the circumferential direction of the cap cover 36. The two second protrusions 58 are provided at positions 180° out of phase in the circumferential direction of the locking portion 38. The first protrusions 56 and the second protrusions 58 face each other in an unopened state of the cap 18 (see Fig. 6A).

Each first protrusion 56 is formed with a first inclined surface 56a inclined in the distal end direction toward one side in the circumferential direction of the cap cover 36 (a direction in which the tightening of the male thread 32 and the female thread 50 is loosened). Each second protrusion 58 is formed with a second inclined surface 58a inclined in the distal end direction toward one side in the circumferential direction of the cap cover 36. The second inclined surface 58a faces the first inclined surface 56a with space therebetween in the unopened state of the cap 18.

The second protrusions 58 are configured as restriction portions to engage the protruding portions 28 in the circumferential direction of the cap cover 36 to restrict the movement of the locking portion 38 in the rotational direction with respect to the cylindrical portion 26.

The prefilled syringe 10 according to the present embodiment is basically configured as above. Next, an opening operation of the cap 18 in the unopened state and a recapping operation of reattaching the cap 18 once removed from the syringe barrel 12 to the syringe barrel 12 will be described. Note that in the unopened state of the cap 18, the side wall 46 of the cylindrical body 37 and the locking portion 38 are connected to each other by the two connecting portions 40 (see Fig. 6A).

When opening the cap 18, the user rotates the cap cover 36 to one side in the circumferential direction of the cap cover 36 with respect to the cylindrical portion 26 of the syringe barrel 12 (the direction in which the tightening of the male thread 32 and the female thread 50 is loosened). Then, the cap cover 36 tends to be displaced in the distal end direction with respect to the cylindrical portion 26 by the action of screws of the female thread 50 and the male thread 32, with the movement of the locking portion 38 in the distal end direction with respect to the cylindrical portion 26 being restricted by the protruding portions 28, so that tensile forces act on the connecting portions 40. Further, shearing forces act on the connecting portions 40 by the protruding portions 28 and the second protrusions 58 contacting each other. Then, the connecting portions 40 are broken by the tensile forces and shearing forces. Subsequently, the cylindrical body 37 and the plug 35 are displaced in the distal end direction with respect to the cylindrical portion 26, and the cylindrical body 37 and the plug 35 are removed from the cylindrical portion 26, whereby the opening operation of the cap 18 is completed (see Fig. 6B). A male luer 102 is connected to the cylindrical portion 26 from which the cylindrical body 37 and the plug 35 have been removed (see Fig. 8).

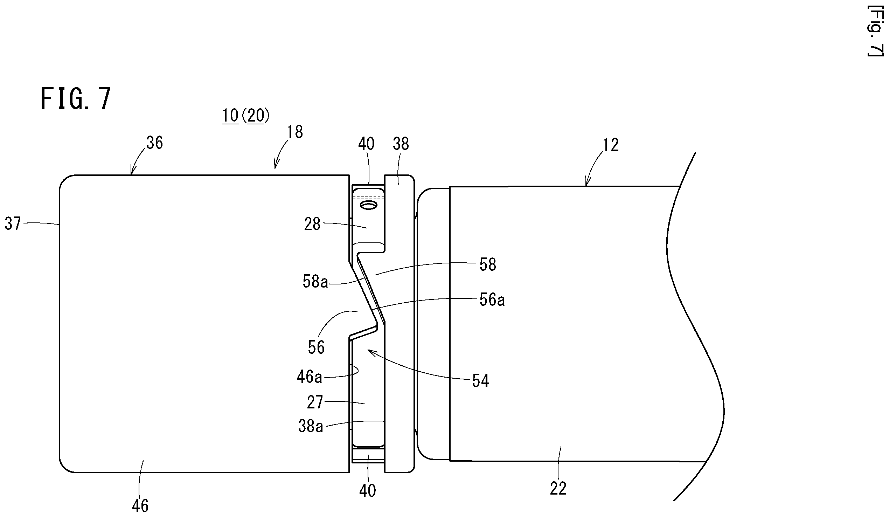

When recapping the opened cylindrical body 37 and the plug 35 to the cylindrical portion 26 of the syringe barrel 12, the user inserts the cylindrical portion 26 into the cylindrical body 37 such that the insertion portion 42 of the plug 35 is inserted into the cylindrical portion 26. Then, the user rotates the cylindrical body 37 to the other side in the circumferential direction of the cap cover 36 with respect to the cylindrical portion 26 (a direction in which the male thread 32 and the female thread 50 are tightened to each other). Then, the cap cover 36 is displaced in the proximal end direction with respect to the cylindrical portion 26 by the action of screws of the male thread 32 and the female thread 50, and the distal-end opening 26a of the cylindrical portion 26 is liquid-tightly sealed by the sealing portion 44, whereby the recapping operation of the cap 18 is completed (see Fig. 7).

Next, the function and effect of the present embodiment will be described below.

In the present embodiment, the connecting portions 40 for opening the cap 18 are broken. Specifically, in the unopened state of the cap 18, the connecting portions 40 are not broken, and in the recapped state in which the cylindrical body 37 and the plug 35 once removed from the cylindrical portion 26 are reattached to the cylindrical portion 26, the connecting portions 40 are broken. This allows easy and reliable discrimination between the unopened state and the recapped state of the cap 18. Further, since the distance L1 from the distal end of the cylindrical portion 26 to the distal end of each protruding portion 28 in the axial direction of the cylindrical portion 26 is set in the range of 5 mm to 10 mm, the protruding portions 28 can be prevented from being obstacles when the male luer 102 of the different syringe 100 is connected to the cylindrical portion 26 configured as the female luer (see Fig. 8). Consequently, the male luer 102 can be securely connected to the cylindrical portion 26.

The protruding portions 28 are located between the cylindrical body 37 and the locking portion 38. Thus, when the cylindrical body 37 is displaced relative to the cylindrical portion 26 in the distal end direction, the locking portion 38 comes into contact with the protruding portions 28, so that the connecting portions 40 can be easily broken.

The two protruding portions 28 are provided at positions 180° out of phase in the circumferential direction of the cylindrical portion 26. The locking portion 38 is annularly formed, and has the inner diameter D1 smaller than the distance L2 between the protruding ends of the protruding portions 28 in the radial direction of the cylindrical portion 26. This allows the locking portion 38 to be reliably brought into contact with the protruding portions 28 when the cylindrical body 37 and the plug 35 are removed from the cylindrical portion 26.

The connecting portions 40 are provided between the two protruding portions 28 in the circumferential direction of the cylindrical portion 26, so that interference between the connecting portions 40 and the protruding portions 28 can be avoided.

The proximal-end-side annular portion 27 is provided on the outer peripheral surface of the cylindrical portion 26, and the protruding portions 28 extend from the proximal-end-side annular portion 27. Thus, the proximal-end-side annular portion 27 can improve the rigidity of the cylindrical portion 26 as the female luer, and can improve the rigidity of the protruding portions 28.

The inner diameter D1 of the locking portion 38 is larger than the outer diameter D2 of the proximal-end-side annular portion 27. Thus, at the time of production of the syringe assembly 20, for example, by elastically deforming the locking portion 38 into an elliptical shape, the minor axis of the ellipse can be made larger than the outer diameter D2 of the proximal-end-side annular portion 27 while the major axis of the ellipse are made larger than the distance L2 between the protruding ends of the protruding portions 28. This allows the locking portion 38 to cross the protruding portions 28 from the distal end direction when the syringe assembly 20 is produced, so that the locking portion 38 can be easily fitted on the proximal end side beyond the protruding portions 28.

The connecting portions 40 are provided in the circumferential direction of the cap cover 36, and thus can facilitate visual recognition of whether the connecting portions 40 are broken.

The thickness dimension of the connecting portions 40 along the radial direction of the cap cover 36 is smaller than the thickness dimension of the side wall 46 of the cylindrical body 37, so that the user can break the connecting portions 40 with a moderate force.

The female thread 50 is formed on the inner peripheral surface of the cylindrical body 37, and the male thread 32 to be screwed to the female thread 50 is provided on the distal end side of the outer peripheral surface of the cylindrical portion 26. Thus, by rotating the cylindrical body 37 relative to the cylindrical portion 26 with the male thread 32 and the female thread 50 screwed together, the cylindrical body 37 can be easily displaced in the distal end direction relative to the cylindrical portion 26.

The locking portion 38 has the second protrusions 58 that engage the protruding portions 28 in the circumferential direction of the cap cover 36 to restrict the movement of the locking portion 38 in the rotational direction with respect to the cylindrical portion 26. Thus, by rotating the cap cover 36 in the circumferential direction with respect to the cylindrical portion 26, the protruding portions 28 and the second protrusions 58 are engaged with each other, so that shearing forces in the circumferential direction can be applied to the connecting portions 40. Therefore, the connecting portions 40 can be easily broken.

The present invention is not limited to the configuration described above. The cap 18 may not be provided with the guide 54. Also, the cap 18 may not be provided with the female thread 50. When the cap 18 is not provided with the female thread 50, rotating the cap cover 36 to one side in the circumferential direction with respect to the locking portion 38 brings the first inclined surfaces 56a and the second inclined surfaces 58a into contact with each other, causing the first protrusions 56 to be guided by the second protrusions 58 in the distal end direction. Thus, the cap cover 36 can be easily displaced in the distal end direction with respect to the cylindrical portion 26.

Further, the cap 18 may not be provided with the guide 54 and the female thread 50. In this case, by pulling the cap cover 36 in the distal end direction with respect to the cylindrical portion 26, the user can break the connecting portions 40 and displace the cap cover 36 in the distal end direction with respect to the cylindrical portion 26.

In addition to the protruding portions 28 and the second protrusions 58, a rotation stop (restriction portion) may be provided which restricts displacement of the locking portion 38 in the circumferential direction with respect to the cylindrical portion 26. The provision of this rotation stop allows the user to rotate the cap cover 36 in the circumferential direction with respect to the cylindrical portion 26, thereby applying shearing forces in the circumferential direction to the connecting portions 40, so that the connecting portions 40 can be easily broken.

Further, one or three or more protruding portions 28 may be provided.

The cap 18 may be attached to the syringe barrel 12 only by the engagement of the locking portion 38 and the protruding portions 28 in the unopened state of the cap 18. In this case, by the connecting portions 40 being broken at the time of opening the cap 18, the cylindrical body 37 and the plug 35 are separated from the locking portion 38. Thus, the cylindrical body 37 and the plug 35 can be prevented from being recapped to the cylindrical portion 26.

The syringe assembly and the prefilled syringe according to the present invention are not limited to the above-described embodiment, and various configurations can be adopted without departing from the scope of the present invention, as a matter of course.

## Claims

1. A syringe assembly (20) comprising: a syringe barrel (12) having at a distal end thereof a cylindrical portion (26) configured as a female luer to which a male luer (102) is connectable; and a cap (18) attached to the syringe barrel (12),
wherein the cap (18) comprises:
a cylindrical cap cover (36); and
a plug (35) fixed inside the cap cover (36), for liquid-tightly sealing an opening (26a) of the cylindrical portion (26),
the cap cover (36) comprises:
a cylindrical body (37) to which the plug (35) is fixed, for covering the cylindrical portion (26);
a locking portion (38) provided on a proximal end side of the cylindrical body (37); and
connecting portions (40) connecting the cylindrical body (37) and the locking portion (38) in a breakable manner,
the cylindrical portion (26) is provided, on an outer peripheral surface thereof, with at least one protruding portion (28) for restricting movement of the locking portion (38) in a distal end direction with respect to the cylindrical portion (26),
a distance (L1) from a distal end of the cylindrical portion (26) to a distal end of the at least one protruding portion (28) in an axial direction of the cylindrical portion (26) is set in a range of 5 mm to 10 mm, and
the cylindrical body (37) and the plug (35) are removed from the syringe barrel (12) by breaking the connecting portions (40).

2. The syringe assembly (20) according to claim 1, wherein
the at least one protruding portion (28) is located between the cylindrical body (37) and the locking portion (38) .

3. The syringe assembly (20) according to claim 2, wherein
the at least one protruding portion (28) comprises two protruding portions (28) provided at positions 180° out of phase in a circumferential direction of the cylindrical portion (26), and
the locking portion (38) is formed in an annular shape, and has an inner diameter (D1) smaller than a distance (L2) between protruding ends of the protruding portions (28) in a radial direction of the cylindrical portion (26).

4. The syringe assembly (20) according to claim 3, wherein
the connecting portions (40) are provided between the two protruding portions (28) in the circumferential direction of the cylindrical portion (26).

5. The syringe assembly (20) according to any one of claims 1 to 4, wherein
the cylindrical portion (26) is provided with an annular portion (27) on the outer peripheral surface thereof, and
the at least one protruding portion (28) extends from the annular portion (27).

6. The syringe assembly (20) according to claim 5, wherein
the locking portion (38) has an inner diameter (D1) larger than an outer diameter (D2) of the annular portion (27) .

7. The syringe assembly (20) according to any one of claims 1 to 6, wherein
the connecting portions (40) are provided in a circumferential direction of the cap cover (36).

8. The syringe assembly (20) according to any one of claims 1 to 7, wherein
the connecting portions (40) have a thickness dimension along a radial direction of the cap cover (36) smaller than a thickness dimension of a side wall of the cylindrical body (37).

9. The syringe assembly (20) according to any one of claims 1 to 8, wherein
the cylindrical body (37) has a female thread (50) formed on an inner peripheral surface thereof, and
the cylindrical portion (26) has a male thread (32) provided on the outer peripheral surface thereof and screwed to the female thread (50).

10. The syringe assembly (20) according to any one of claims 1 to 9, wherein
the locking portion (38) has a restriction portion (58) that is engaged with the at least one protruding portion (28) in the circumferential direction of the cap cover (36), for restricting movement of the locking portion (38) in a rotational direction with respect to the cylindrical portion (26).

11. The syringe assembly (20) according to claim 10, wherein
the cap (18) is attached to the syringe barrel (12) only by engagement of the locking portion (38) with the at least one protruding portion (28), in an unopened state of the cap (18).

12. The syringe assembly (20) according to any one of claims 1 to 11, wherein
the cap (18) has a guide (54) for guiding the cylindrical body (37) in the distal end direction when the cylindrical body (37) is rotated relative to the cylindrical portion (26).

13. A prefilled syringe (10) comprising:
the syringe assembly (20) according to any one of claims 1 to 12;
a medicine (M) with which the syringe barrel (12) of the syringe assembly (20) is filled; and
a gasket (14) slidable in the syringe barrel (12) liquid-tightly in an axial direction.
